(19) Europäisches Patentamt
European Patent Office
Office européen des brevets

(11) **EP 0 973 443 B1**

(12) **EUROPÄISCHE PATENTSCHRIFT**

(45) Veröffentlichungstag und Bekanntmachung des Hinweises auf die Patenterteilung:
**22.03.2006 Patentblatt 2006/12**

(21) Anmeldenummer: **98901970.8**

(22) Anmeldetag: **15.01.1998**

(51) Int Cl.:
*A61M 16/00* (2006.01)    *A61M 16/12* (2006.01)
*B01F 3/02* (2006.01)     *A61M 16/10* (2006.01)

(86) Internationale Anmeldenummer:
**PCT/EP1998/000202**

(87) Internationale Veröffentlichungsnummer:
**WO 1998/031282 (23.07.1998 Gazette 1998/29)**

(54) **GESTEUERTES GASVERSORGUNGSSYSTEM**

CONTROLLED GAS-SUPPLY SYSTEM

SYSTEME D'ALIMENTATION EN GAZ COMMANDE

(84) Benannte Vertragsstaaten:
**AT BE CH DE DK ES FI FR GB GR IE IT LI LU MC NL PT SE**

(30) Priorität: **17.01.1997 DE 19701617**
**23.10.1997 DE 19746742**

(43) Veröffentlichungstag der Anmeldung:
**26.01.2000 Patentblatt 2000/04**

(73) Patentinhaber: **INO-Therapeutics GmbH**
**1111 Wien (AT)**

(72) Erfinder: **KREBS, Christian**
**A-1120 Wien (AT)**

(74) Vertreter: **Kasseckert, Rainer et al**
**Linde Aktiengesellschaft,**
**Zentrale Patentabteilung**
**Dr.-Carl-von-Linde-Strasse 6-14**
**82049 Höllriegelskreuth (DE)**

(56) Entgegenhaltungen:
| | |
|---|---|
| **EP-A- 0 354 388** | **EP-A- 0 413 555** |
| **EP-B- 0 073 219** | **DE-A- 3 416 291** |
| **DE-A- 3 708 146** | **DE-A- 3 712 598** |
| **DE-A- 4 309 923** | **DE-C- 4 325 319** |
| **DE-C- 4 327 730** | **GB-A- 2 170 731** |
| **US-A- 4 686 974** | **US-A- 4 928 684** |
| **US-A- 4 971 049** | **US-A- 5 195 528** |

## Beschreibung

Die Erfindung betrifft ein Gerät zur gesteuerten Dosierung von Gasen, insbesondere für die kontrollierte Zudosierung von NO oder Sauerstoff zu einem Atemgas bei Geräten zur Beatmung oder Atemspende.

Geräte zur Beatmung werden eingesetzt für die maschinelle Beatmung, für die Anästhesie und für die Atemtherapie durch Behandlung mit Gasen, beispielsweise Sauerstoffspende oder Behandlung mit Stickstoffmonoxid (NO).

Ein Inhalations-Anästhesiegerät wird beispielsweise in der DE-A 37 12 598 beschrieben; es dient zur Dosierung von Narkosegas in das Atemgas.

Die DE-C 43 25 319 beschreibt ein Gerät zur kontinuierlichen Dosierung von NO zur Atemluft von Patienten, enthaltend einen Respirator, einen. NO-Dosierbehälter, eine Dosiereinheit mit Steuergerät und einen Analysator zur Bestimmung der NO-Konzentration in der Atemluft. Das Steuergerät (Kontroll- und Regeleinheit) übernimmt die Dosierung des zu dosierenden NO durch Bestimmung der Volumenströme von Atemgas und NO und Berücksichtigung des NO-Analysewertes. Die NO-Dosierung erfolgt volumenproportional oder Volumenstromproportional, so dass die NO-Konzentration im Atemgas immer konstant gehalten wird. Die technischen Grundzüge der NO-Dosierung in der NO-Therapie werden beschrieben in: "C. Krebs et al., Technological Basis for NO Application and Environment Security, Acta Anaesthesiologica Scandinavica Supplement 109, Vol. 40, 1996, S.84-87"

Aus der US-A 4 928 684 ist ein Gasversorgungssystem zur Unterstützung der Spontanatmung mit einer sensorgesteuerten Dosierung eines Luft/Sauerstoff-Gasgemisches offenbart. Hierbei ist die sensorgesteuerte Dosierung zwar inspirationssynchronisiert, eine pulsmodulierte oder sequenzielle Dosierung des Luft/Sauerstoff-Gasgemisches ist jedoch nicht möglich.

Des Weiteren wird in der US-A 4 971 049 eine Verfahrensweise beschrieben, gemäß der auf ein Triggersignal hin zu einem Atemgas Sauerstoff in Form eines Pulses mit einstellbarer Pulsweite zudosiert wird. Nicht offenbart wird jedoch, dass bei der Zudosierung von Sauerstoff zu dem Atemgas eine Pulsmodulation oder Dosierungssequenz realisiert werden könnte.

Patienten mit chronischen Atembeschwerden (z. B. Asthma und COPD (Chronisch obstruktive Atemwegserkrankung / Chronic Obstructive Pulmonary Disease)) werden durch einen in der Regel transportablen Sauerstoffspender in der Sauerstoffversorgung des Körpers unterstützt. Solche Patienten werden als spontanatmende Patienten bezeichnet, im Unterschied zu Patienten, die in der Klinik intubiert an ein Beatmungsgerät angeschlossen sind. Spontanatmende Patienten erhalten so z. B. eine zusätzliche Sauerstoffspende (LOT = Langzeitsauerstofftherapie) oder eine Atmungsunterstützung (CPAP = Continuous Positive Airways pressure). Verabreicht werden die Gase entweder über eine so genannte Nasenbrille oder Nasensonde (Nasalapplikation; im einfachsten Fall ein Gasversorgungsschlauch, dessen Öffnung unterhalb der Nasenöffnungen des Patienten offen angeordnet ist) oder per Atemmaske (besonders bei CPAP).

Ein Gerät zur Zufuhr von Atemgas oder Sauerstoff zu einem Patienten wird auch in der DE-A 43 09 923 beschrieben. Ein Pulsoxymeter dient dabei zum Anpassen des dem Patienten zuzuführenden Atemgasvolumens an den ermittelten Blutgassättigungsgrad.

Der Erfindung liegt die Aufgabe zugrunde, ein Gerät für die Versorgung von Patienten mit einem oder mehreren Gasen zu schaffen, wobei die Gasdosierung in ein Atemgas durch eine Steuerung des Gerätes auf einen Patienten individuell abgestimmt ist. Insbesondere soll die dem Patienten verabreichte Gasmenge gesenkt bzw. optimiert werden.

Gelöst wird diese Aufgabe durch ein Gasversorgungssystem mit den Merkmalen der Ansprüche 1 oder 2.

Gasversorgungssysteme sind Anordnungen oder Vorrichtungen, einem Patienten ein oder mehrere Gase zuführen oder für die Atmung bereitstehen. Die Gase, insbesondere medizinische Gase, werden vorzugsweise mit Luft, einem Atemgas oder Sauerstoff gemischt, so dass Gasgemische erhalten werden, die die Atmung unterhalten. Ein Gasversorgungssystem ist z. B. ein Beatmungssystem mit Beatmungsgerät und Gasdosiervorrichtung für ein oder mehrere Gase. Ein Beatmungssystem besteht beispielsweise aus Schlauchverbindungen oder Gasleitungen, Gasquelle, Gasdosiervorrichtung (Gasdosiereinheit), Atemmaske, vorzugsweise einem Atemgasanfeuchter und gegebenenfalls einem oder mehreren Gasfiltern (z. B. $NO_2$-Filter). Beatmungssysteme mit einem Beatmungsgerät werden im allgemeinen für die stationäre Behandlung von Patienten eingesetzt. Gasversorgungssysteme können stationäre oder mobile, insbesondere tragbare Geräte sein. Gasversorgungssysteme gemäß der Erfindung dienen bevorzugt zur Behandlung von Mensch oder Säugetier mit einem oder mehreren Gasen, insbesondere zur inhalativen Behandlung der Lunge.

Ein Beatmungssystem, das mit Änderungen als Gasversorgungssystem eingesetzt werden kann, ist beispielsweise in DE 43 25 319 C1 beschrieben, worauf Bezug genommen wird.

Gasversorgungssysteme sind beispielsweise auch gasspendende Geräte für spontanatmende Patienten. Solch ein Gasversorgungssystem ist in DE 43 09 923-A1 beschrieben, worauf Bezug genommen wird.

Das Gasversorgungssystem enthält in der Regel eine Atemmaske oder eine Nasenbrille. Das Gasversorgungssystem enthält vorzugsweise einen Befeuchter für das Atemgas und/oder Gas.

Das Gasversorgungssystem enthält vorzugsweise eine oder mehrere Gasquellen. Gasquellen sind beispielsweise Druckgasquellen mit einem komprimierten Gas wie Druckgasbehälter, Druckgasflaschen, Druckdosen, Druckgasleitungen oder Behälter mit kälteverflüssigtem Gas (z. B. zur Abgabe von verdampftem, gasförmigem Sauerstoff).

Als Gasquelle kann auch ein Gaserzeuger dienen. Ein Gaserzeuger ist beispielsweise ein onsite-Gasgenerator, z. B. zur Erzeugung von Stickstoffmonoxid (NO), insbesondere NO in Stickstoff, durch Glimmentladung in einem Stickstoff/ Sauerstoff-Gasgemisch. Weitere Gaserzeuger sind z. B. Elektrolysezellen (z. B. zur Erzeugung von Wasserstoff) oder chemische Reaktoren (z. B. Reaktionskammern mit chemischen Reaktionen zur Gaserzeugung).

Die Gase sind vorzugsweise medizinische Gase. Medizinische Gase sind Gase oder Gasgemische, die im Bereich der Medizin eingesetzt werden, z. B. zur Behandlung von Krankheiten; Therapie, Prophylaxe, Narkose, Diagnostik, Verbesserung der Atemfunktion oder des Befindens bei Mensch oder Säugetier. Medizinische Gase haben oft eine pharmakologische Wirkung. Medizinische Gase können aber auch aufgrund anderer Eigenschaften eingesetzt werden (z. B. als Kontrastmittel für die Tomographie, insbesondere NMR-Computertomographie der Lunge oder andere bilderzeugende Verfahren). Medizinische Gase sind z. B. Sauerstoff, Narkosegase wie Lachgas ($N_2O$) oder Xenon, Wasserstoff, Edelgase wie Helium, Kohlendioxid ($CO_2$), Stickstoffmonoxid (NO) oder Gasgemische mit einem oder mehreren der genannten Gase als Bestandteil, z. B. Helium/Sauerstoff-Gasgemische, Helium/Sauerstoff/NO-Gasgemische oder Helium/Sauerstoff/$CO_2$-Gasgemische. Statt der Dosierung eines Gasgemisches können auch die einzelnen Komponenten oder einzelne Komponenten und Teilgasgemische nebeneinander (zeitgleich oder zeitversetzt) z. B. einem Atemgas zudosiert werden. Medizinische Gase haben im allgemeinen eine hohe Reinheit.

Die Dosierung eines oder mehrerer Gase erfolgt vorteilhaft nur während der Phasen des Einatmens (Inspiration). Während des Ausatmens (Exspiration) erfolgt keine Gasdosierung. Eine auf die Atemzyklen synchronisierte Gasdosierung wird durch eine Triggerung mit Hitfe eines Sensors erreicht. Aufgrund von Sensormeßwerten wird der Beginn der Inspiration oder der Beginn und das Ende der Inspiration von einer Steuereinheit erkannt. Die Gasdosierung erfolgt kontinuierlich (z. B. mit fest vorgegebener Menge oder Konzentration des dosierten Gases pro Inspiration über die gesamte Betriebszeit) oder diskontinuierlich (z. B. mit Dosierpausen), vorzugsweise

    a) programmgesteuert (z. B. Zeitprogramm),
    b) sensorgesteuert oder
    c) mit einer kombinierten Programmsteuerung und Sensorsteuerung.

Die Steuereinheit (z. B. Mikroprozessor-, Computer-Steuerung) empfängt das Meßsignal des Sensors zur Triggerung der Gasdosierung und bestimmt vorzugsweise aufgrund einer Programm- und/oder Sensor-Steuerung, ob das Gas zudosiert wird, über welche Zeitdauer (Pulsbreite $t_i$), welchen Volumenstrom $V_i'$ (differentielle Änderung des Gasvolumens $V_i$ zur Zeit t: $V_i'=dV_i/dt$=Pulshöhe) und welche Anzahl $n_i$ von Zudosierungen ($n_i$: Zahl der Impulse) die Dosierung des Gases i erfolgt. Diese Art der Gasdosierung wird pulsmodulierte Gasdosierung genannt. Die Zeitdauer $t_{max}$ der Inspiration bzw. Beginn und Ende der Inspiration wird vorteilhaft mittels eines Sensors bestimmt. Die Pulsbreite $t_i$ ist kleiner oder gleich der Zeitdauer $t_{max}$. Das dosierte Gasvolumen $V_i$ eines Impulses berechnet sich nach $V_i= V_i' * t_i$, das während einer Inspiration dosierte Gasvolumen nach $V_i= V_i' * t_i * n_i$.

Die Konzentration $C_i$ eines dosierten Gases, bezogen auf das Atemgasvolumen $V_{ges}$ ($V_{ges}$=Summe aller Gasvolumina $V_i$), berechnet sich bei $n_i$=1 nach

$$C_i = V_i / V_{ges} = V_i' * t_i / V_{ges}.$$

Die Werte der Pulsbreite, Pulshöhe und Zahl der Impulse innerhalb eines Atemzuges (Inspiration) können fest voreingestellt oder variabel sein.

Die Dosierung eines Gases wird bei vielen Anwendungen vorteilhaft durch die Kombination einer Grunddosierung, vorzugsweise mit konstanten Einstellungen von $V_i'$, $n_i$ und $t_i$, und einer additiven Dosierung mit variablen (gesteuerten) Einstellungen von $V_i'$, $n_i$ und $t_i$ vorgenommen. Grunddosierung und additive Dosierung eines Gases erfolgen bevorzugt mit getrennten Dosiereinrichtungen (z. B. gesteuerte Magnetventile). Die Grunddosierung kann dabei eine Grundversorgung mit einem Gas sicherstellen und die Regelung der Gasmenge und der Gaskonzentration durch die additive Gasdosierung erreicht werden. In diesem Fall kann die additive Gasdosierung programm- oder sensorgesteuert erfolgen.

Zur Steuerung der Dosierung eines Gases können Meßwerte des vorangegangenen Atemzuges dienen, z. B. die Dauer der Inspiration ($t_{max}$) und/oder das Atemgasvolumen ($V_{ges}$). Regelgrößen sind z. B. die Gaskonzentration C, oder das Mischungsverhältnis von Gasen (z. B. $V_1/V_2$).

Die Gasdosierung kann mittels eines Programms zwischen einem unteren und oberen Grenzwert verändert werden, z. B. kann die Gaskonzentration über eine Folge von Atemzügen erhöht und gesenkt werden (z. B. in regelmäßiger Folge bei gleichem oder ungleichem Verhältnis von Erhöhung und Senkung der Gaskonzentration; vorteilhaft bei NO-Dosierung). Die Gasdosierung kann auch vorteilhaft nach einer zuvor am Patienten ermittelten Response-Kurve gesteuert werden. Zur Ermittlung der Response-Kurve wird mit einem Sensor ein Körperparameter des Patienten (z. B.

Sauerstoffsättigung im peripheren Blut und/oder Herzfrequenz, mittels Pulsoxymeter erfaßt) in Abhängigkeit von der dosierten Gasmenge oder Gaskonzentration gemessen und der zeitliche Bedarf an Gas zur Einstellung eines gleichmäßigen Körperzustandes bestimmt.

Der Ablauf des Programms zur Steuerung der Dosierung eines Gases ist bei einer weiteren Verfahrensvariante der Gasdosierung von dem Erreichen bestimmter Meßgrößen, die von einem oder mehreren Sensoren erfaßt werden, abhängig. Beispielsweise kann das über- oder unterschreiten eines Schwellenwertes einer Meßgröße Programmabläufe der Gasdosierung auslösen. Durch einen Schwellenwert kann ein Programmteil aktiviert werden, der einen Dosierablauf für hohe, mittlere oder niedrige Gasdosierung bewirkt.

Die Gasdosierung ist vorteilhaft eine Zudosierung des Gases (z. B. Sauerstoff oder NO oder NO-haltiges Gas) zum Atemgas in Dosierintervallen einer bestimmten Folge (sequentielle Gasdosierung). So erfolgt die Gasdosierung z. B. durch eine sich wiederholende Folge von Atemzyklen (Inspirationen) mit in das Atemgas zudosiertem Gas (Gaszudosierung) und Atemzyklen ohne Zudosierung von Gas (Auslassung).

Die sequentielle Gasdosierung ist beispielsweise eine Wiederholung der folgenden Sequenzen (regelmäßige Sequenzen) bei gleicher Dauer der Dosierintervalle (z. B. Sauerstoff- oder NO-Zudosierung bei der Beatmung oder bei spontanatmenden Patienten):

a) eine Gaszudosierung und eine Auslassung,
b) 2 Gaszudosierungen (d. h. 2 Inspirationsphasen mit Gaszudosierung) und 25 folgende Auslassungen (d. h. 25 folgende Inspirationsphasen ohne Gaszudosierung),
c) 10 Gaszudosierungen und 30 folgende Auslassungen oder
d) 3 Gaszudosierungen und 80 folgende Auslassungen.

Eine Gasdosierung kann auch aus variablen (unregelmäßigen) Sequenzen bestehen, z. B. eine Abfolge von Sequenzen mit zunehmender oder abnehmender Zahl von Gasdosierungen.

Die einfachste Sequenz ist die Folge von einer Gaszudosierung und einer Auslassung. Die Wiederholung der Sequenz ergibt den Gesamtablauf der Dosierschritte (der Gasdosierung). Beispiele für verschiedene Formen der sequentiellen Gasdosierung sind in der Tabelle aufgeführt.

Tabelle: Ausführungsformen der sequentiellen Gasdosierung (mit $n_i$=1)

| Art der Dosierung | Gaskonzentration oder Gasmenge im Dosierintervall | Zeitdauer des Dosierintervalles | Sequenz der Dosierintervalle |
| --- | --- | --- | --- |
| 1. | konstant | konstant | konstant |
| 2. | konstant | konstant | variabel |
| 3. | konstant | variabel | konstant |
| 4. | konstant | variabel | variabel |
| 5. | variabel | variabel | konstant |
| 6. | variabel | konstant | variabel |
| 7. | variabel | konstant | konstant |
| 8. | variabel | variabel | variabel |

Die sequentielle Gasdosierung hat den Vorteil, daß ein Gas zeitweise hoch dosiert werden kann und trotzdem im zeitlichen Mittel eine sehr niedrige Gaskonzentration oder Gasmenge eingesetzt wird. Beispielsweise kann über eine Folge von einem oder mehreren (zwei, drei, vier, fünf, sechs, sieben, acht, neun, zehn oder mehr) Atemzügen eine übliche NO-Dosis (z. B. bis zu 80 ppm NO im Atemgas bei schwerstem Lungenversagen) und dann über eine Folge von Atemzügen (ein, zwei, drei, vier, fünf, sechs, sieben, acht, neun, zehn oder zwanzig, dreißig, vierzig, fünfzig oder mehr Atemzüge) eine sehr niedrige NO-Menge verabreicht werden, so daß sich eine durchschnittliche NO-Konzentration z. B. im ppb- oder ppt-Bereich ergibt. Die sequentielle Gasdosierung von zwei, drei oder mehreren Gasen kann kombiniert werden.

Die gesteuerte Gasdosierung führt zu einem geringereren Gasverbrauch, insbesondere zu einer geringeren verabreichten Gesamt-Gasmenge, wodurch Nebenwirkungen des Gases (z. B. NO) beim Patienten reduziert werden können. Als weiterer Vorteil werden Absetzung und Entwöhnung der Gas-Therapie (z. B. NO-Therapie) erleichtert. Allgemein bei der Entwöhnung beatmeter NO-pflichtiger Patienten ist eine stetige Verminderung der NO-Gabe vorteilhaft. Bei der Verwendung eines Beatmungssystems mit gesteuerter NO-Dosierung ist ein weiterer wichtiger Vorteil eine insgesamt geringere, aus NO gebildete Menge von toxischem $NO_2$ bei der Beatmung.

Regelorgane für die Gasdosierung sind vorteilhaft elektrisch steuerbar. Als Regelorgane dienen z. B. zeit- und/ oder sensorgesteuerte Magnetventile (z. B. Magnetventil mit vorgeschalteter Elektronik der Firma Bürkert, Deutschland), Massendurchflußregler (z. B. Gerätetyp MFC der Firma Brooks, Niederlande), automatisch einstellbare Druckregler (z. B. mittels Schrittmotor oder Elektromotor verstellbar) oder Regelventile zur direkten, insbesondere automatischen Verstellung des Gasdruckes. Bei Gasquellen mit kälteverflüssigtem Gas wird vorteilhaft die Verdampfung des Gases mittels einer Heizvorrichtung im Vorratsbehälter reguliert. Die Heizvorrichtung ist vorzugsweise eine elektrische Widerstandsheizung, die durch Ein-/Aus-Schaltung des Heizstromes oder durch kontinuierliche Veränderung der Heizleistung gesteuert wird. Zusätzlich kann das Gas über ein Magnetventil in der Gasversorgungsleitung dosiert werden.

Sensoren sind allgemein Meßfühler. Der Begriff umfaßt auch (im weiteren Sinne) Meßgeräte und Analyseeinrichtungen. Die Verwendung der Sensoren teilt sich auf in Sensoren zur Auslösung oder Triggerung der Gasdosierung (Auslöse-Sensoren), Sensoren zur Steuerung des Verlaufes der Gasdosierung (Regel-Sensoren) und Sensoren zur Sicherheitsheitsüberwachung des Gasversorgungssystems (z. B. zum Auslösen von Alarm oder zur Sicherheitsabschaltung von Gerätefunktionen, insbesondere mittels Gassensoren).

Als Auslöse-Sensor eignet sich ein Drucksensor, der den Gasdruck mißt, insbesondere ein Unterdrucksensor. Das Meßsignal des Sensors kann bereits als Steuersignal dienen (z. B. bei sogenanntem "Smart Sensor") oder mittels einer elektronischen Verarbeitungs- und Steuereinrichtung in ein Steuersignal umgewandelt werden. Der Sensor kann beispielsweise den Druck (Gasdruck) in oder vor der Nase messen (z. B. durch einen in die Atemmaske oder Nasenbrille integrierten Sensor). Ein Drucksensor eignet sich auch zur Erfassung des Verlaufs des inspiratorischen Unterdrucks und kann zur Steuerung einer bedarfsangepaßten Gasdosierung eingesetzt werden (z. B. höhere Gasdosierung bei tiefen Atemzügen, geringere Gasdosierung bei flacher Atmung). Es können auch Differenzdrücke gemessen und zur Steuerung genutzt werden (z. B. Differenzdruck zu Druck bei entsprechender Phase von vorangegangenem Atemzug), da diese bei definiertem Einstellen den quadratischen Wert des Durchflusses anzeigen.

Regel-Sensoren sind beispielsweise Drucksensoren, gasspezifische Sensoren oder Gassensoren (z. B. elektrochemische Gassensoren für $O_2$, NO oder $NO_2$) und insbesondere Sensoren zur Erfassung von Körperreaktionen, Körperfunktionen oder Körperzuständen des Patienten (patientenbezogene Meßwerte), Sensoren zur Messung der Sauerstoffsättigung im peripheren Blut, z. B. Pulsoxymeter (z. B. Gerät ASAT der Firma Baxter, USA), Sensoren zur Blutgasanalyse (z. B. Gerät 995 HO der Firma AVL, Österreich; Gerät "Perotrend" der Firma Crosstec), Sensoren zur Messung von Blutdruck oder Sensoren für Lungenblutdruck (auch Pulmonaldruck oder Lungenarteriendruck; mittels eines in die Lungenarterie eingeschwemmten Katheders, z. B. Type SWAN-Ganz von Baxter, USA, mit elektrischer Umsetzung mittels des Gerätes "Explorer" von Baxter), Sensoren zur Messung von Herzzeitvolumen oder Herzfrequenz oder Sensoren zur Erfassung von Beatmungsparametern wie Beatmungsdruck, Beatmungsvolumen oder Compliance (Dehnbarkeit der Lunge).

Herzfrequenz und Sauerstoffsättigung im peripheren Blut können mittels Pulsoxymeter gemessen werden. Die simultane Erfassung beider Größen wird vorteilhaft zur Steuerung der Gasdosierung eingesetzt, z. B. bei der Sauerstoffdosierung und/oder NO-Dosierung in Beatmungssystemen oder Systemen für spontanatmende Patienten, insbesondere bei der Gastherapie von COPD-Patienten.

Vorzugsweise werden stark miniaturisierte Sensoren (insbesondere Drucksensoren) eingesetzt, die eine direkte Plazierung, am Meßort (z. B. an der Nase, am oder im Körper des Patienten) ermöglichen. Der Sensor kann aber auch von dem eigentlichen Meßort entfernt angeordnet sein, z. B. in der Dosierleitung plaziert oder durch eine geeignete Schlauchleitung mit dem Meßort verbunden. Dies kann beispielsweise bei Vakuummeßgeräten (Druckmeßgeräten) als Sensor der Fall sein oder bei Sensoren (Meßgeräten, Analysegeräten) zur Bestimmung der Konzentration einer Gaskomponente, z. B. NO-, Kohlendioxid- oder Sauerstoffkonzentration. Es können zur Steuerung der Gasdosierung und/ oder Gasmischung auch verschiedene Sensoren kombiniert werden. Beispielsweise können Drucksensoren und Gassensoren kombiniert eingesetzt werden.

Der Einsatz der Sensoren erlaubt eine automatische, patientenbezogene Gasdosierung.

Im folgenden wird die Erfindung anhand der NO-Dosierung, der Sauerstofftherapie und der kombinierten Dosierung von NO-haltigem Gas und Sauerstoff erläutert.

Die Steuerung der NO-Dosierung erfolgt vorteilhaft anhand einer Response-Kurve des Patienten auf NO. Es wird die Response-Kurve des Patienten vorher bestimmt, das heißt die zeitliche Abhängigkeit einer Meßgröße (eines Parameters) von der verabreichten NO-Menge oder NO-Konzentration. Die Response-Kurve kann beispielsweise durch Messung der durch die NO-Dosierung bewirkte steigende Sauerstoffsättigung im peripheren Blut und/oder durch den bei der NO-Dosierung fallenden Pulmonaldruck ermittelt werden. Anhand dieser Response-Kurve kann die günstigste NO-Dosierung bestimmt werden. Zur Steuerung der NO-Dosierung kann ein empirisch bestimmter Sollwert mit der Meßgröße vergüchen werden und dementsprechend eine Regeleinheit (z. B. Durchflußregler oder Magnetventil) angesteuert werden, wobei z. B. die NO-Menge so gesteuert wird, daß die zeitliche Änderung der online gemessenen Meßgröße der Response-Kurve angenähert wird.

Grenzwerte der einzustellenden NO-Konzentration (minimale, maximale Konzentration), Atemzyklenzahl mit und ohne Zudosierung und optimale Parameter für die Steuerung der Gasdosierung können in einer vorangegangenen Bestimmung oder während der eigentlichen Therapie bestimmt werden (Bestimmung der Steuerungsparameter: zeitlich gewünschter Gaskonzentrationsverlauf). Zur Optimierung der NO-Dosierung (automatische Findung und Anpassung der günstigsten (minimal erforderlichen) NO-Menge kann folgendermaßen vorgegangen werden: 1. stetige NO-Mengensteigerung (NO-Zunahme) von Untergrenze (z. B. 0,1ppm NO) bis Obergrenze (z. B. 100 ppm), dabei Messung der Sauerstoffsätigung im peripheren Blut und/oder der Pulmonaldruck (Beobachtung der Reaktion des Patienten = Response). Bestimmung der günstigen NO-Konzentration (wird Sollwert für Steuerung). Kontrolle des Sollwertes mit zweiter Response-Messung (Durchfahren der NO-Konzentration Untergrenze/Obergrenze/Untergrenze = Dreiecksmessung). Das optimale NO-Profil (NO-Konzentrationskurve im Atemgas) ist dann erreicht, wenn sich eine gleichbleibende Sauerstoffsättigung im peripheren Blut oder ein minimaler, konstanter Pulmonaldruck einstellt (adaptive Steuerung der Gasdosierung).

Das Gasversorgungssystem wird beispielsweise bei der Behandlung von Hypoxie oder Lungenhochdruck mit NO verwendet. Eine Verwendung erfolgt vorteilhaft auch bei folgenden Krankheiten/Krankheitsbildern: ARDS (adult respiratory distress syndrome), Asthma, PPH (angeborener Lungenhochdruck), COPD (chronisch obstruktive Lungenerkrankung), Herzmißbildung, Lungenunreife bei Früh- und Neugeborenen.

Bei Gasversorgungssystemen für die Sauerstofftherapie wird vorteilhaft die Messung der Sauerstoffsättigung von Hämoglobin im peripheren Blut (z. B. Messung mittels eines Pulsoxymeter) genutzt. Die Sauerstoffkonzentration im Atemgas oder die Sauerstoffmenge werden gesteuert. Der Regelbereich der Sauerstoffkonzentration reicht bis 100 Vol.-%. Analog zu der NO-Zudosierung wird bei diesem Verfahren, bei dem das Gasversorgungssystem eingesetzt wird, die Sauerstoff-Zudosierung gezielt geregelt.

Bei der Sauerstofftherapie können diskontiniuerlichen Meßverfahren zur Erfassung der Oxygenierung im Blutkreislauf als Meß- und Regelgröße, z. B. mit dem Gerät 995 HO der Firma AVL (Österreich), oder kontinuierliche Meßverfahren, z. B. mit dem Gerät "Perotrend" der Firma Crosstec, eingesetzt werden. Die Blutgasanalyse erfaßt in der Regel arterielles Blutgas, venöses Blutgas oder gemischt-venöses Blutgas.

Das Gasversorgungssystem zur automatischen Sauerstoff-Dosierung bei der Sauerstofftherapie eignet sich vorteilhaft zum Einsatz sowohl bei spontanatmenden wie auch bei beatmeten Patienten. Insbesondere eine pulsmodulierte Gasdosierung von Sauerstoff oder anderen zusätzlichen Gasen wird vorteilhaft anhand von Meßgrößen wie Sauerstoffgehalt des Blutes und/oder der Lungenblutdruck oder Sauerstoffgehalt des Blutes und/oder Herzfrequenz gesteuert.

Eine Programmsteuerung der Sauerstoffdosierung und gegebenenfalls weiterer dosierter Gase erlaubt einen besonders einfachen Aufbau eine Gasversorgungssystems, insbesondere eine transportablen Gasversorgungssystems für chronisch kranke Patienten (z. B. COPD-Patienten).

Besonders vorteilhaft eignet sich das Gasversorgungssystem für eine gesteuerte, abgestimmte Gasdosierung von zwei oder mehreren Gasen, z. B. ausgewählt aus den Gasen NO, Sauerstoff, Wasserstoffgas, Helium und Kohlendixid. Die gezielte Dosierung von Helium dient der Verbesserung der Belüftung der Lunge, Kohlendioxid regt die Atmung an. Für jedes Gas kann eine Sensorsteuerung und/oder eine Programmsteuerung vorgesehen werden. Die Dosierung von zwei oder mehreren Gasen kann anhand der Ermittlung von Gesamtvolumsstrom, oder Teilvolumsströme der einzelnen Gase erfolgen. Die Dosierung der Gase kann prinzipiell wie bei der Dosierung eines einzelnen Gases erfolgen. Bevorzugt wird eine aufeinander abgestimmte Dosierung der Gase. Beispielsweise kann das Mischungsverhältnis der Gase als Steuerparameter gewählt werden. Bei der Dosierung mehrerer Gase können für die einzelnen Gase natürlich unterschiedliche Steuerungsarten eingesetzt werden, z. B. ein Teil der Gase mit Sensorsteuerung, ein Teil mit Programmsteuerung oder ein Teil mit kombinierter Programm-/Sensorsteuerung. Durch geeignete Wahl einer oder mehrerer Gasquellen (z. B. Flüssigsauerstoff, NO-haltiges Gas, insbesondere NO-haltiges Gas aus einem onsite-Generator) und angepaßte Steuerung kann die zum Betrieb des Gasversorgungssystems erforderliche Energie stark reduziert werden (vorteilhaft bei Mobilsystemen mit Batteriebetrieb).

Bei der pulsmodulierten Gasdosierung, insbesondere bei der Dosierung von zwei oder mehreren Gasen, ist eine möglichst homogene Vermischung des Atemgases wichtig, um Konzentrationsspitzen eines Gases im Atemgas zu vermeiden. Vorteilhaft wird eine Homogenisierung des Gasgemisches durch einen Mischkörper im Schlauchsystem, bevorzugt im Atemschlauch, erzielt. Vorzugsweise wird Hohlzylinderteil, das ein spiralförmig verdrehtes Teil (z. B. Metallband oder Kunststoffband mit 180° zueinander verdrehten Enden) als Mischkörper enthält, in das Schlauchsystem eingebaut.

Die Mischstrecke wird sowohl für Schlauchsysteme im Intensivbereich (22 mm Schlauchdurchmesser für Erwachsenen-, 15 mm für Kinder-, 10 mm für Neonatenbeatmung) als auch für z. B. 8 mm- bzw. 10 mm-Schlauchsysteme für die Heimtherapie von chronisch Erkrankten, insbesondere COPD-Patienten.

Auch Filter, Absorber oder Befeuchter , z. B. in einem Atemschlauch, verbessern die Homogenität der Mischung von Gasen.

Der patientennahe Einsatz bei der NO-Applikation, z. B. bei chronischen Erkrankungen, wird durch Einsatz eines Filters für Stickstoffdioxid ($NO_2$), z. B. Filter mit Polyphenylensulfid als Filtermaterial oder Natriumcarbonat-Patrone (Sodalime), verbessert. Z. B. kann auch ein onsite-Generator für NO mit einem $NO_2$-Filter vorteilhaft kombiniert werden. Die folgenden Figuren erläutern die Erfindung und beschreiben Gasversorgungssysteme für spontanatmende Patienten (z. B. COPD-Patienten).

Fig. 1 zeigt schematisch eine Atemmaske 2 mit Sensor 1 (z. B. Drucksensor) und Gasversorgungsschlauch 3 (z. B. Sauerstoff) als Teile eines Gaseversorgungssystems.

Fig. 2 zeigt schematisch eine Nasenbrille 4 mit Sensor 1 (z. B. Drucksensor) und Gasversorgungsschlauch 3 (z. B. Sauerstoff). Zur Versorgung des Patienten mit verschiedenen Gasen können mehrere Nasenbrillen 4 am Patienten angeordnet werden. Alternativ zu mehreren Nasenbrillen sind koaxiale Schläuche möglich, bei denen durch jedes Lumen ein anderes Gas strömt.

Fig. 3 zeigt schematisch ein Diagramm des Nasendruckes $P_N$ als Funktion der Zeit t ohne Gasdosierung, gemessen mit einem Drucksensor vor der Nasenöffnung. Die Marken a und b zeigen Start und Ende eines Inspirationsintervalles (Einatmen).

Fig. 4 zeigt schematisch ein Diagramm des gemessenen Nasendruckes $P_N$ als Funktion der Zeit t bei Dosierung von Sauerstoff. Das untere Diagramm (Schema) zeigt den Volumenstrom von dosiertem Sauerstoff im Dosierintervall a bis b (Inspirationsintervall).

Fig. 5 zeigt schematisch ein Diagramm des gemessenen Nasendruckes $P_N$ als Funktion der Zeit t bei getakteter Dosierung von Sauerstoff. Das untere Diagramm (Schema) zeigt den Volumenstrom von getaktetem dosiertem Sauerstoff im Dosierintervall a bis b (Inspirationsintervall).

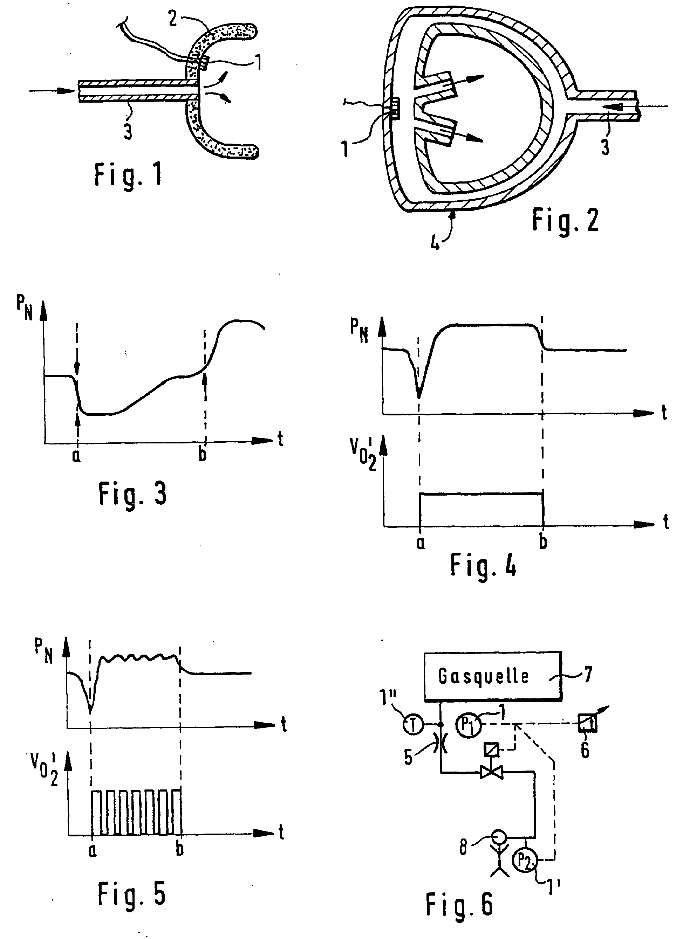

Fig. 6 zeigt schematisch eine sensorgesteuertes Gasversorgungssystem mit mehreren Sensoren 1 (P1: Druck), 1' (P2: Druck) und 1" (T: Temperatur) und einer Gasquelle 7 (z. B. Sauerstoff), Ist der Druck des Gases (z. B. Sauerstoff) entweder durch eine einmalige oder eine kontinuierliche Messung des Druckes (P1) sowie der Durchmesser einer oder möglicherweise mehrerer Düsen 5 oder Einengungen (kann auch z. B. der Durchmesser des Ventileinganges oder Ventilsitzes sein) bekannt, so kann einmalig oder kontinuierlich der zum Patienten applizierte Volumsstrom und durch Kenntnis der Zeitdauer das applizierte Gasvolumen ermittelt werden. Es ist auch möglich, mittels Temperatur (Temperatursensor) den Volumsstrom über eine Druck-/Temperatur-Rückrechnung den genauen Normvolumsstrom zu ermitteln. Der Auslöser des Beginnes der Inspirationsphase und damit der Beginn der Öffnung des Magnetventiles kann durch den Unterdrucksensor P2 getriggert werden. Durch ein an einem Potentiometer an der Steuereinheit 6 zugeordnetes Volumen (oder aber Eingabe/Anzeige eines höher elektronifizierten Systemes wie z.B. Mikroprozessor/Controller) wird die Zeitdauer der Öffnung und damit das zu applizierende Volumen angezeigt bzw. eingestellt.

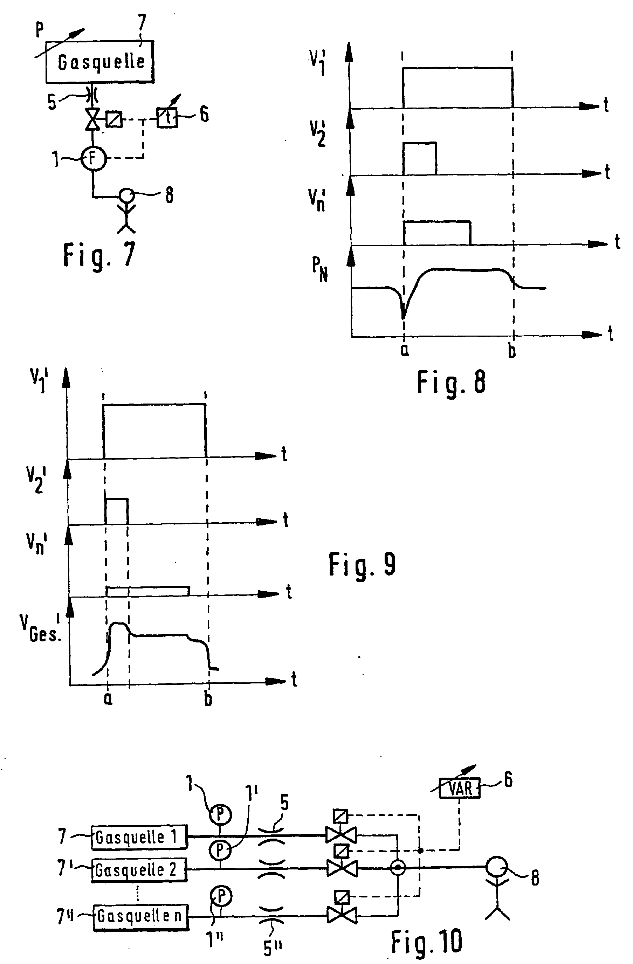

Fig. 7 zeigt schematisch ein Gasversorgungssystem mit Druckreduziereinrichtung an der Gasquelle 7. Durch Variierung des Druckes des Versorgungsgases, dies kann ein Druckgasbehälter mit Druckreduziereinrichtung oder ein Flüssiggasbehälter mit Verdampfungseinrichtung mit oder ohne Druckreduziereinrichtung sein, kann der Volumenstrom verändert werden. Dies wird durch die Druckmessung erkannt und die neue Zeit oder aber das neue applizierte Volumen kann dargestellt und berechnet/gesteuert werden.

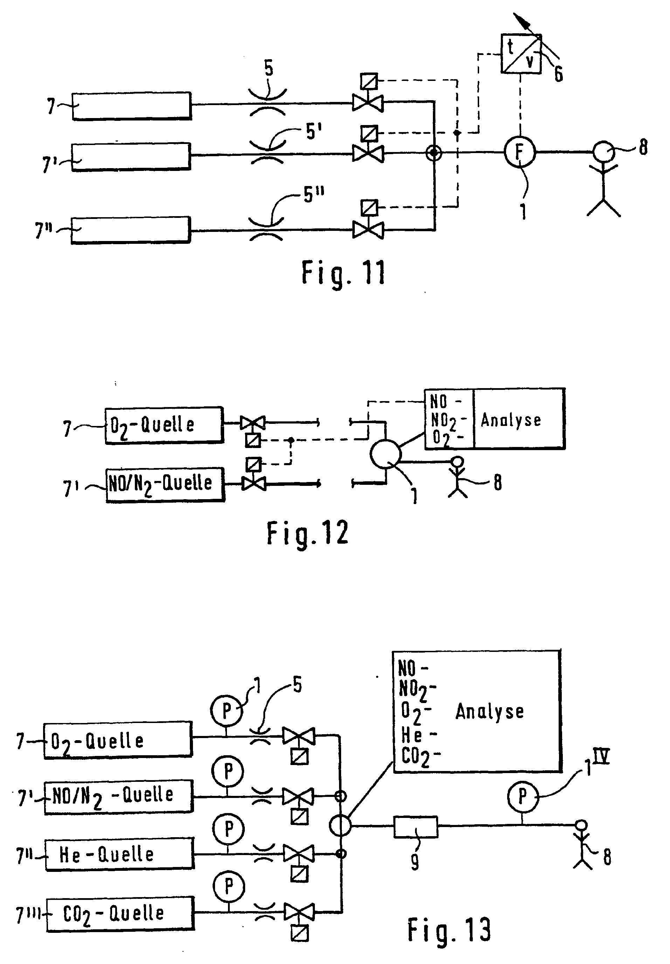

Fig. 8 zeigt schematisch den Verlauf des Gesamtvolumenstromes analog dem Nasendruck $P_N$ (unterstes Diagramm) bei Dosierung mehrerer Gase mit den jeweiligen Volumenströmen $V_1$, $V_2$, $V_n$. Eine geeignetes Gasversorgungssystem ist in Fig. 11 gezeigt.

Fig. 9 zeigt analog zu Fig. 8 den Verlauf von erzeugten Volumenströmen verschiedener Gase. Fig. 8 und 9 sind Beispiele von verschiedenen, erzeugten Mischungsverhältnissen mehrerer Gase.

Fig. 10 zeigt schematisch ein Gasversorgungssystem mit mehreren Gasquellen 7, 7' und 7" und zugeordneten Drucksensoren 1, 1' und 1".

Fig. 11 zeigt schematisch ein Gasedosiersystem mit mehreren Gasquellen 7, 7' und 7" und zugeordneten Druckreduziereinrichtungen (z. B. Düsen) 5, 5' und 5" und einem Sensor 1 zur Steuerung der Magnetventile über eine Steuereinheit 6.

In Fig. 12 wird die Abgabe von Gas aus den Gasquellen 7 (z. B. Sauerstoff) und 7' (z. B. NO-Quelle) über einen Sensor 1 und/oder eine Gasanalyseeinheit gesteuert.

Fig. 13 zeigt ein Gasversorgungssystem mit mehreren Gasquellen 7 bis 7''' (z. B. Sauerstoff, NO-Quelle, Helium, Kohlendioxid) mit Sensoren 1 bis 1''' und patientennahem Sensor 1[IV] und Filterelement 9.

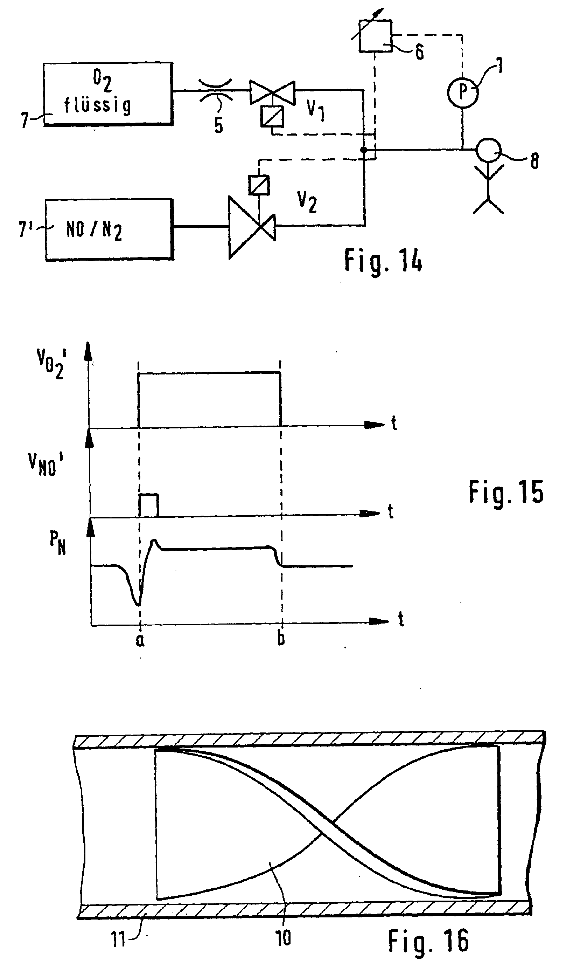

Fig. 14 zeigt schematisch ein Gasversorgungssystem für Flüssigsauerstoff und NO-haltiges Gas. Die Ventile V1 und V2 (z. B. Magnetventile) werden über den patientennahen Sensor 1 (z. B. Drucksensor) in Verbindung mit der Steuereinheit 6 geregelt.

Fig. 15 zeigt schematisch den zeitlichen Verlauf Volumenströme von Sauerstoff und NO-haltigem Gas und des gemessenen Nasendruckes $P_N$.

Fig. 16 zeigt eine Mischvorrichtung für Gase aus Hohlzylinderteil 11 und Mischkörper 10, der durch einen verdrehten Flachkörper (z. B. aus Metall, Kunstoff oder Glas; Verdrehung der Enden des Flachkörpers zueinander: z. B. 180° oder 360°) gebildet wird. Die Mischvorrichtung wird als Mischstrecke bevorzugt in den Atemschlauch des Gasversorgungssystems eingebaut.

Beispiel

Sauerstoffvolumenabhängige NO-Dosierung

Ein tragbares Gerät für die kombinierte Dosierung von Sauerstoff und NO (in Stickstoff) enthält einen Vorratsbehälter für kälteverflüssigten Sauerstoff mit integriertem Verdampfungssystem (Fassungsvermögen: 0,5 Liter), einen Druckgasbehälter für NO-Stickstoff-Gasgemisch (typisch 800 bis 1000 ppm NO in $N_2$; geometrisches Flaschenvolumen: 0,2 bis 1,0 Liter; Fülldruck: 150 bis 200 bar), ein Steuergerät für die Dosierung von Sauerstoff und NO-Gasgemisch, mindestens 2 elektrisch steuerbare Magnetventile, Gasschläuche und Nasenbrille mit Drucksensor, NO-Sensor und $NO_2$-Sensor in der Atemgasleitung, ein Alarmsystem und Sicherheitseinrichtung (Alarm: bei leerer NO-Gasgemisch-Flasche, bei leerem Sauerstoffvorratsbehälter, zu hoher Sauerstoff-, NO- oder $NO_2$-Konzentration im Atemgas).

Der Drucksensor dient zur Triggerung der Gasdosierung (inspirationssynchronisierte Gasdosierung). Bei Beginn der Inspiration werden das Magnetventil für die Sauerstoff-Dosierung und das Magnetventil für die NO-Gasgemisch-Dosierung geöffnet. Der Wert des pro Inspiration dosierten Sauerstoffvolumens ist vorgegeben, z. B. $V_{O2}$= 50 ml. Bei einem eingestellten Sauerstoff-Volumenstrom $V_{O2}'$ von 3000 ml/Minute ergibt sich eine Pulsbreite $t_{O2}$ von 1 Sekunde. Die einzustellende NO-Konzentration im Atemgasvolumen $V_{ges}$ ($V_{ges} = V_{O2} + V_{NO}$) soll $C_{NO}$= 35 ppm betragen. Das NO-Gasgemisch enthält 1000 ppm NO. Der voreingestellte NO-Gasgemisch-Volumenstrom $V_{NO}'$ beträgt 500 ml/Minute. Das erforderliche NO-Gasgemisch-Dosiervolumen $V_{NO}$ zur Einstellung der NO-Konzentration $C_{NO}$= 35 ppm (Volumen/Volumen) im Atemgas berechnet sich folgendermaßen:

Es gilt $C_{NO}=(V_{NO} * F) / V_{ges} = (V_{NO} * F) / (V_{O2} + V_{NO})$
mit F: NO-Konzentration im NO-Gasgemisch.

Daraus folgt $V_{NO} = (V_{O2} * C_{NO}) / (F-C_{NO})$.

Mit $V_{O2} = 3000$ ml, $C_{NO} = 35$ (ppm) und F=1000 (ppm) ist $V_{NO}$ =1,8 ml. Durch $V_{NO} = V_{NO}' * t_{NO}$ ist die Öffnungszeit des NO-Dosierventils (hier Funktion auf/zu) festgelegt. Die Öffnungszeit des Magnetventils für NO-Gasgemisch-Dosierung beträgt daher 218 Millisekunden (bei $V_{NO}'$ = 500 ml/Minute). Aus Homogenitätsgründen ist es vorteilhaft, die Dosierbedingungen so zu wählen, daß die NO-Dosierzeit bei $t_{NO}$ =½ $t_{O2}$ liegt. Dies wird erreicht durch Verringerung des Volumenstroms $V_{NO}'$ durch Senkung des Vordrucks in der Gasdosierleitung. Die Senkung des Gäsvordrucks erfolgt vorteilhaft durch eine in die Gasdosierleitung eingebaute steuerbare Blende oder Düse (automatische Verstellung der Blendenöffnung oder Düsenöffnung).

Zur einfacheren Darstellung enthält das Berechnungsbeispiel nur eine vorgegebene NO-Konzentration. Durch ein Steuerprogramm oder eine Sensorsteuerung wird bevorzugt die NO-Konzentration variiert.

**Patentansprüche**

1. Gasversorgungssystem zur inhalativen Behandlung von Mensch oder Säugetier mit einer gesteuerten Gasdosierung von einem oder mehreren therapeutisch wirksamen Gasen, aufweisend einen Auslöse sensor zur inspirationssynchronisierten Triggerung und eine Steuereinheit, wobei die Steuereinheit ein Messsignal des Sensors zur Triggerung der Gasdosierung empfängt und wobei das Gas nur während des Inhälationszyklus zudosiert wird, **dadurch gekennzeichnet, dass** aufgrund einer Programm- und/oder Sensorsteuerung bestimmt wird, ob Gas zudosiert wird, und wenn Gas zudosiert wird, über welche Zeitdauer mit welchem Volumenstrom und mit welcher Anzahl von Zudosierungen die Dosierung des Gases erfolgt.

2. Gasversorgungssystem zur inhalativen Behandlung von Mensch oder Säugetier mit einer gesteuerten Gasdosierung von einem oder mehreren therapeutisch wirksamen Gasen aufweisend einen Auslöse sensor zur inspirationssynchronisierten Triggerung und eine Steuereinheit, wobei die Steuereinheit ein Messsignal des Sensors zur Triggerung der Gasdosierung empfängt und wobei das Gas nur während des Inhalafionszyklus zudosiert wird, **dadurch gekennzeichnet, dass** eine Programmsteuerrung, eine Sensorsteuerung oder eine Kombiniente Programm-/Sensorsteuerung vorgesehen ist, die Gasdosierung durch eine sich wiederholende Folge von Atemzyklen mit in das Atemgas zudosiertem Gas und Atemzyklen ohne Zudosierung von Gas durchzuführen.

3. Gasversorgungssystem nach einem der vorhergehenden Ansprüche 1 bis 2, **dadurch gekennzeichnet, dass** das Gasversorgungssystem Mittel aufweist, die eine Grunddosierung und eine additive Dosierung des oder der dosierten Gase ermöglichen.

4. Gasversorgungssystem nach einem der vorhergehenden Ansprüche 1 bis 3, **dadurch gekennzeichnet; dass** das Gasversorgungssystem einen Druckbehälter mit komprimiertem Gas, einen Behälter mit kälteverflüssigtem Gas oder einen Gaserzeuger als Gasquelle (7, 7', 7", ....) enthält.

5. Gasversorgungssystem nach Anspruch 4, **dadurch gekennzeichnet, dass** die Gasquelle (7, 7', 7", ....) eine Gasquelle (7; 7', 7", ....) ist

- für Sauerstoff und ein NO-haltiges Gas;
- für ein NO-haltiges Gas und Wasserstoff;
- für Sauerstoff und Wasserstoff;
- für Sauerstoff und Helium;
- für Sauerstoff, ein NO-haltiges Gas und Wasserstoff;
- für Sauerstoff, ein NO-haltiges Gas und Helium;
- für Sauerstoff, Kohlendioxid und Helium oder
- für Sauerstoff, ein NO-haltiges Gas, Kohlendioxid und Wasserstoff.

6. Gasversorgungssystem nach einem der vorhergehenden Ansprüche 1 bis 5, **dadurch gekennzeichnet, dass** das Gasversorgungssystem einen gasspezifischen Sensor, einen Gassensor, einen Gasanalysator, einen Drucksensor (1, 1'), einen Drucksensor (1, 1') und einen Gassensor, einen Schallsensor einen Schalldrucksensor, einen Gasvolumenstromsensor, einen Gasvolumensensor, einen Sensor zur Messung der Sauerstoffsättigung im peripheren Blut, zur Blutgasanalyse, zur Messung von Blutdruck, zur Messung von Lungenblutdruck, zur Messung von Herzfrequenz oder zur Messung von Herzzeitvolumen enthält.

7. Gasversorgungssystem nach einem der vorhergehenden Ansprüche 1 bis 6, **dadurch gekennzeichnet, dass** das Gasversorgungssystem ein oder mehrere Sensoren (1, 1', 1") zur simultanen Messung von mindestens zwei Messgrößen enthält, wobei die Messgrößen zur Steuerung der Gasdosierung dienen.

8. Gasversorgungssystem nach einem der vorhergehenden Ansprüche 1 bis 7, **dadurch gekennzeichnet, dass** das Gasversorgungssystem ein oder mehrere Sensoren zur simultanen Messung von Herzfrequenz und Sauerstoffsättigung im peripheren Blut oder von Lungengefäßdruck und Sauerstoffsättigung im peripheren Blut enthält.

9. Gasversorgungssystem nach einem der vorhergehenden Ansprüche 1 bis 8, **dadurch gekennzeichnet, dass** das Gasversorgungssystem eine auf den behandelten Menschen oder das behandelte Säugetier bezogene oder adaptive Steuerung (6) der Gasdosierung von mindestens einem Gas enthält.

10. Gasversorgungssystem nach einem der vorhergehenden Ansprüche.1 bis 9, **dadurch gekennzeichnet, dass** das Gasversorgungssystem einen Sensor für ein Alarmsystem oder zur Steuerung einer Sicherheitsabschaltung einer Gasdosierung enthält.

11. Gasversorgungssystem nach einem der vorhergehenden Ansprüche 1 bis 10, **dadurch gekennzeichnet, dass** Gasversorgungssystem eine Programmsteuerung (6) und/oder Sensorsteuerung (6) zur kontinuierlichen oder diskontinuierlichen zeitlichen Änderung der Gasmenge oder Gaskonzentration eines Gases im Atemgas enthält.

12. Gasversorgungssystem nach einem der vorhergehenden Ansprüche 1 bis 11, **dadurch gekennzeichnet, dass** das Gasversorgungssystem nach der Einspeisung eines oder mehrerer dosierter Gase eine Mischstrecke und/oder einen Mischkörper in der Atemgasleitung enthält.

**Claims**

1. Gas supply system for treating humans or mammals by inhalation with a controlled metering of one or more therapeutically active gases, which includes a release sensor for inspiration-synchronized triggering and a control unit, the control unit receiving a measurement signal from the sensor in order to trigger the gas metering, and the gas being metered in only during the inhalation cycle, **characterized in that** a programme and/or sensor control unit is used to determine whether gas is being metered in, and if gas is being metered in the duration for which and also the volumetric flow with which and the number of metered doses in which the gas is metered.

2. Gas supply system for treating humans or mammals by inhalation with a controlled metering of one or more therapeutically active gases, which includes a release sensor for inspiration-synchronized triggering and a control unit, the control unit receiving a measurement signal from the sensor in order to trigger the gas metering, and the gas being metered in only during the inhalation cycle, **characterized in that** a programme control unit, a sensor control unit or a combined programme/sensor control unit is provided, which is suitable for carrying out the gas metering through a recurring sequence of breathing cycles with gas metered into the respiratory gas and breathing cycles without gas metered in.

3. Gas supply system according to one of the preceding Claims 1 to 2, **characterized in that** the gas supply system includes means which allow base-level metering and additive metering of the metered gas(es).

4. Gas supply system according to one of the preceding Claims 1 to 3, **characterized in that** the gas supply system includes a pressure vessel containing compressed gas, a vessel containing cryogenically liquefied gas or a gas generator as gas source (7, 7', 7'', ...).

5. Gas supply system according to Claim 4, **characterized in that** the gas source (7, 7', 7'', ...) is a gas source (7, 7', 7'', ...)

   - for oxygen and an NO-containing gas;
   - for an NO-containing gas and hydrogen;
   - for oxygen and hydrogen;
   - for oxygen and helium;
   - for oxygen, an NO-containing gas and hydrogen;
   - for oxygen, an NO-containing gas and helium;
   - for oxygen, carbon dioxide and helium or
   - for oxygen, an NO-containing gas, carbon dioxide and hydrogen.

6. Gas supply system according to one of the preceding Claims 1 to 5, **characterized in that** the gas supply system contains a gas-specific sensor, a gas sensor, a gas analyser, a pressure sensor (1, 1'), a pressure sensor (1, 1') and a gas sensor, a sound sensor, a sound pressure sensor, a gas volumetric flow sensor, a gas volume sensor, a sensor for measuring the oxygen saturation in the peripheral blood, for blood gas analysis, for measuring blood pressure, for measuring pulmonary blood pressure, for measuring heart rate or for measuring cardiac output.

7. Gas supply system according to one of the preceding Claims 1 to 6, **characterized in that** the gas supply system contains one or more sensors (1, 1', 1'') for simultaneously measuring at least two variables, the variables being used to control the gas metering.

8. Gas supply system according to one of the preceding Claims 1 to 7, **characterized in that** the gas supply system contains one or more sensors for simultaneously measuring heart rate and oxygen saturation in the peripheral blood or pulmonary vessel pressure and oxygen saturation in the peripheral blood.

9. Gas supply system according to one of the preceding Claims 1 to 8, **characterized in that** the gas supply system contains a control unit (6), which is oriented or adaptive with respect to the treated person or mammal, for the metering of at least one gas.

10. Gas supply system according to one of the preceding Claims 1 to 9, **characterized in that** the gas supply system contains a sensor for a warning system or for controlling a safety shut-off for gas metering.

11. Gas supply system according to one of the preceding Claims 1 to 10, **characterized in that** the gas supply system contains programme control unit (6) and/or sensor control unit (6) for the continuous or discontinuous temporal change in the gas volume or gas concentration of a gas in the respiratory gas.

12. Gas supply system according to one of the preceding Claims 1 to 11, **characterized in that** the gas supply system, after one or more metered gases have been fed in, contains a mixing path and/or a mixing member in the respiratory gas line.

**Revendications**

1. Système d'alimentation en gaz pour le traitement par inhalation de l'homme ou d'un mammifère, doté d'un dispositif asservi d'addition dosée d'un ou de plusieurs gaz à action thérapeutique qui présente un détecteur de déclenchement et une unité de commande, l'unité de commande recevant un signal de mesure du détecteur de déclenchement de l'addition dosée de gaz synchronisée par rapport à l'inspiration, le gaz n'étant ajouté que pendant le cycle d'inhalation, **caractérisé en ce que** sur base d'une commande programmée et/ou d'une commande par détecteur, on détermine si du gaz est ajouté et s'il en est ajouté, pendant quelle durée, à quel débit volumique et le nombre des additions dosées du gaz réalisées.

2. Système d'alimentation en gaz pour le traitement par inhalation de l'homme ou d'un mammifère, doté d'un dispositif asservi d'addition dosée d'un ou de plusieurs gaz à action thérapeutique qui présente un détecteur de déclenchement et une unité de commande, l'unité de commande recevant un signal de mesure du détecteur de déclenchement de l'addition dosée de gaz synchronisée par rapport à l'inspiration, le gaz n'étant ajouté que pendant le cycle d'inhalation, **caractérisé en ce qu'**une commande par programme, une commande par détecteur ou une commande combinée par programme et par détecteur est prévue et permet de réaliser l'addition dosée de gaz par une succession répétée de cycles de respiration avec addition de gaz dans le gaz respiré et de cycles de respiration sans addition dosée de gaz.

3. Système d'alimentation en gaz selon les revendications 1 ou 2 qui précèdent, **caractérisé en ce que** le système d'alimentation en gaz présente des moyens qui permettent une addition dosée de base et une addition dosée supplémentaire du ou des gaz ajoutés.

4. Système d'alimentation en gaz selon les revendications 1 à 3 qui précèdent, **caractérisé en ce que** le système d'alimentation en gaz contient comme source de gaz (7, 7', 7'', ...) un réservoir sous pression qui contient du gaz comprimé, un réservoir qui contient un gaz liquéfié à froid ou un générateur de gaz.

5. Système d'alimentation en gaz selon la revendication 4, **caractérisé en ce que** la source de gaz (7, 7', 7'', ...) est une source de gaz (7, 7', 7'', ...) qui fournit :

   - de l'oxygène ou un gaz qui contient du NO,
   - un gaz qui contient du NO et de l'hydrogène,
   - de l'oxygène et de l'hydrogène,
   - de l'oxygène et de l'hélium,
   - de l'oxygène, un gaz qui contient du NO et de l'hydrogène,
   - de l'hydrogène, un gaz qui contient du NO et de l'hélium,
   - de l'oxygène, du dioxyde de carbone et de l'hélium ou
   - de l'oxygène, un gaz qui contient du NO, du dioxyde de carbone et de l'hydrogène.

6. Système d'alimentation en gaz selon l'une des revendications 1 à 5 qui précèdent, **caractérisé en ce que** le système d'alimentation en gaz contient une sonde spécifique au gaz, une sonde de gaz, un analyseur de gaz, une sonde de pression (1, 1'), une sonde de pression (1, 1') et une sonde de gaz, une sonde acoustique, une sonde de pression acoustique, une sonde de débit volumique de gaz, une sonde de volume de gaz, une sonde de mesure de la saturation en oxygène du sang périphérique, pour l'analyse des gaz présents dans le sang, pour la mesure de la pression sanguine, pour la mesure de la pression sanguine dans les poumons, pour la mesure de la fréquence cardiaque ou pour la mesure du volume par pulsation cardiaque.

7. Système d'alimentation en gaz selon l'une des revendications 1 à 6 qui précèdent, **caractérisé en ce que** le système d'alimentation en gaz contient plusieurs sondes (1, 1', 1'') pour la mesure simultanée d'au moins deux grandeurs de mesure, les grandeurs de mesure servant à commander l'addition dosée de gaz.

8. Système d'alimentation en gaz selon l'une des revendications 1 à 7 qui précèdent, **caractérisé en ce que** le système d'alimentation en gaz contient une ou plusieurs sondes pour la mesure simultanée de la fréquence cardiaque et de la saturation en oxygène du sang périphérique ou de la pression des vaisseaux dans les poumons et la saturation en oxygène dans le sang du système périphérique.

9. Système d'alimentation en gaz selon l'une des revendications 1 à 8 qui précèdent, **caractérisé en ce que** le système d'alimentation en gaz contient une commande (6) adaptative ou adaptée à la personne traitée ou au mammifère traité de l'addition dosée d'au moins un gaz.

10. Système d'alimentation en gaz selon l'une des revendications 1 à 9 qui précèdent, **caractérisé en ce que** le système d'alimentation en gaz contient une sonde pour un système d'alarme ou pour la commande d'un débranchement de sécurité de l'addition dosée de gaz.

**11.** Système d'alimentation en gaz selon l'une des revendications 1 à 10 qui précèdent, **caractérisé en ce que** le système d'alimentation en gaz contient une commande par programme (6) et/ou une commande par sonde (6) pour modifier en continu ou de manière discontinue dans le temps la quantité ou la concentration d'un gaz dans le gaz respiré.

**12.** Système d'alimentation en gaz selon l'une des revendications 1 à 11 qui précèdent, **caractérisé en ce que** le système d'alimentation en gaz contient en aval de l'addition dosée d'un ou de plusieurs gaz un parcours de mélange et/ou un corps de mélange dans le conduit de gaz respiré.

Fig. 1

Fig. 2

Fig. 3

Fig. 4

Fig. 5

Fig. 6

Fig. 7

Fig. 8

Fig. 9

Fig. 10

Fig. 11

Fig. 12

Fig. 13

Fig. 14

Fig. 15

Fig. 16